(19) Europäisches Patentamt
European Patent Office
Office européen des brevets

(11) **EP 2 912 000 B1**

(12) **EUROPÄISCHE PATENTSCHRIFT**

(45) Veröffentlichungstag und Bekanntmachung des Hinweises auf die Patenterteilung:
**01.03.2017 Patentblatt 2017/09**

(21) Anmeldenummer: **13770841.8**

(22) Anmeldetag: **28.09.2013**

(51) Int Cl.:
*C07C 29/141* (2006.01)   *C07C 45/75* (2006.01)
*B01J 23/86* (2006.01)

(86) Internationale Anmeldenummer:
**PCT/EP2013/002922**

(87) Internationale Veröffentlichungsnummer:
**WO 2014/067600 (08.05.2014 Gazette 2014/19)**

(54) **VERFAHREN ZUR HERSTELLUNG VON NEOPENTYLGLYKOL**

METHOD FOR THE PRODUCTION OF NEOPENTYL GLYCOL

PROCÉDÉ DE PRÉPARATION DE NÉOPENTYLGLYCOL

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.10.2012 DE 102012021280**

(43) Veröffentlichungstag der Anmeldung:
**02.09.2015 Patentblatt 2015/36**

(73) Patentinhaber: **OXEA GmbH**
**46147 Oberhausen (DE)**

(72) Erfinder:
• **EISENACHER, Matthias**
**46485 Wesel (DE)**
• **SCHALAPSKI, Kurt**
**46147 Oberhausen (DE)**
• **STRUTZ, Heinz**
**47445 Moers (DE)**

(56) Entgegenhaltungen:
EP-A1- 0 301 853     WO-A1-98/29374
DE-A1- 1 518 784     GB-A- 2 482 887
US-A- 4 855 515

**Beschreibung**

Die vorliegende Erfindung betrifft ein Verfahren zur Herstellung von Neopentylglykol durch Hydrierung von Hydroxypivalinaldehyd in der Flüssigphase an barium- und mangandotierten Kupferchromit-Katalysatoren.

Mehrwertige Alkohole oder Polyole besitzen als Kondensationskomponente zum Aufbau von Polyestern oder Polyurethanen, Kunstharzlacken, Schmiermitteln und Weichmachern eine erhebliche wirtschaftliche Bedeutung. Hierbei sind besonders solche mehrwertigen Alkohole von Interesse, die durch eine gemischte Aldoladdition von Formaldehyd mit iso- oder n-Butyraldehyd erhalten werden. Bei der Aldoladdition zwischen Formaldehyd und dem entsprechenden Butyraldehyd bildet sich zunächst eine aldehydische Zwischenstufe, die anschließend zum mehrwertigen Alkohol reduziert werden muss. Ein technisch bedeutender mehrwertiger Alkohol, der nach diesem Verfahren zugänglich ist, ist Neopentylglykol [NPG, 2,2-Dimethylpropandiol-(1,3)] aus der Mischaldolisierung von Formaldehyd und iso-Butyraldehyd.

Die Aldoladdition wird in Gegenwart basischer Katalysatoren beispielsweise Alkalihydroxiden oder aliphatischen Aminen durchgeführt und ergibt zunächst das isolierbare Zwischenprodukt Hydroxypivalinaldehyd (HPA). Dieses Zwischenprodukt kann anschließend mit überschüssigem Formaldehyd entsprechend der Cannizzaro-Reaktion in Neopentylglykol unter Bildung eines Äquivalents eines Formiatsalzes überführt werden. Bei dieser Ausgestaltung des Reduktionsschrittes fällt daher das Formiatsalz als Koppelprodukt an und die Wirtschaftlichkeit dieser Verfahrensvariante hängt auch von den Vermarktungschancen des Formiatsalzes ab. Technisch umgesetzt wird aber auch die katalytische Hydrierung von Hydroxypivalinaldehyd in der Gas- und Flüssigphase am Metallkontakt. Als geeignete, Hydrierkatalysatoren haben sich gemäß EP 0 278 106 A1 Nickel-katalysatoren erwiesen. Katalysatoren auf Basis von Kupfer, Zink und Zirkonium werden in dem Verfahren nach EP 0 484 800 A2 in der Hydrierstufe eingesetzt.

Auch Kupferchromit-Katalysatoren werden häufig zur Hydrierung von Hydroxypivalinaldehyd verwendet. Kupferchromit-Katalysatoren enthalten als Aktivatoren häufig noch weitere Metalle, beispielsweise Barium, Cadmium, Magnesium, Mangan und/oder ein seltenes Erdmetall. Gemäß US 4,855,515 zeichnen sich besonders mangandotierte Kupferchromit-Katalysatoren bei der Hydrierung des Aldolisierungsproduktes aus der Umsetzung von Formaldehyd mit iso-Butyraldehyd aus. WO98/29374 A1 offenbart die Verwendung eines bariumdotierten Kupferchromit-Katalysators für die Hydrierung von Hydroxypivalinaldehyd in einer methanolischen Lösung.

Nach der Lehre von DE 1 518 784 A1 wird ein Gemisch aus Hydroxypivalinaldehyd und überschüssigem iso-Butyraldehyd zu Neopentylglykol und iso-Butanol in Gegenwart eines Kupferchromit-Katalysators hydriert, der mit Barium dotiert ist. Auch das zweistufige Hochdruckhydrierverfahren von rohem Hydroxypivalinaldehyd nach EP 0 006 460 A1, bei dem mit ansteigenden Hydriertemperaturen gearbeitet wird, verwendet einen mit Barium aktivierten Kupferchromit-Katalysator.

GB 2482 887 A offenbart die Verwendung von Kupferchromit-Katalysatoren, die sowohl mit Mangan als auch mit Barium dotiert sind, für die Hydrierung von Furfural. Je nach Wahl der Hydriertemperaturen kann die Produktverteilung in Richtung Furfurylalkohol oder 2-Methylfuran gesteuert werden.

In EP 0 301 853 A1 werden Kupferchromit-Katalysatoren beschrieben, die als weitere Zusatzstoffe sowohl Barium als auch Mangan enthalten können. Die bekannten Katalysatoren können für die Hydrierung von Aldehyden verwendet werden.

Die aus dem Stand der Technik bekannten Kupferchromit-Katalysatoren arbeiten bei vergleichsweise hohen Hydriertemperaturen.

Kupferchromit-Katalysatoren besitzen nicht nur eine gute Hydrieraktivität gegenüber Hydroxypivalinaldehyd zum Neopentylglykol sondern sie sind ebenfalls ausreichend aktiv, um Nebenprodukte, die bei der Aldolisierung von iso-Butyraldehyd mit Formaldehyd gebildet werden, unter hydrierenden Bedingungen zu spalten und in den Nebenprodukten gebundenes Neopentylglykol freizusetzen. Solche Nebenprodukte sind beispielsweise Neopentylglykol-iso-butyrat oder der durch Disproportionierung von Hydroxypivalinaldehyd gebildete Tischtschenko-Ester Neopentylglykolmonohydroxypivalinsäureester. Die gezielte hydrierende Spaltung von hochsiedenden Nebenprodukten aus der Neopentylglykolherstellung unter Einsatz von Kupferchromit-Katalysatoren wird in DE 10 2008 033163 A1 beschrieben.

Aus EP 0 522 368 A1 ist bekannt, die Hydrierung von Hydroxypivalinaldehyd in einer Lösung vorzunehmen, die mindestens 20 Gew.-% eines niederen Alkohols beispielsweise Methanol oder n-Butanol, bezogen auf die Mischung aus Alkohol und Reaktionsprodukt, sowie Wasser in einer Menge von nicht mehr als 40 Gew.-%, bezogen auf die Gesamtmenge aus Wasser, Alkohol und Reaktionsprodukt, enthält. Als Hydrierkatalysator wird ein Kupferchromit-Katalysator empfohlen.

Neopentylglykol besitzt als technisch hergestelltes Produkt eine große wirtschaftliche Bedeutung und es besteht daher ein Bedarf, die bekannten Verfahren zur Neopentylglykolherstellung stets zu verbessern, sei es durch eine verbesserte Produktausbeute, durch eine bessere Anlagenausnutzung oder durch einen geringeren Energieeinsatz.

Es wurde überraschenderweise gefunden, dass Neopentylglykol durch Hydrierung von Hydoxypivalinaldehyd in hoher Selektivität und hoher Raum-Zeit-Ausbeute hergestellt werden kann, wenn die Hydrierung in flüssiger Phase in Gegenwart eines Kupferchromit-Katalysators erfolgt, der sowohl mit Mangan als auch mit Barium dotiert ist.

Die vorliegende Erfindung betrifft daher ein Verfahren zur Herstellung von Neopentylglykol durch Addition von iso-Butyraldehyd und Formaldehyd in Gegenwart eines tertiären Alkylamins als Katalysator zum Hydroxypivalinaldehyd mit nachfolgender Hydrierung bei einer Temperatur von 80 bis 140°C und bei einem Druck von 2 bis 18 MPa in der flüssigen Phase, dadurch gekennzeichnet, dass die Hydrierung in Gegenwart eines Kupferchromit-Katalysators enthaltend die Aktivatoren Barium und Mangan erfolgt.

Überraschenderweise wurde gefunden, dass bei Verwendung von Kupferchromit-Katalysatoren, die sowohl Barium als auch Mangan als Aktivatoren enthalten, bei deutlich niedrigeren Temperaturen bereits eine vollständige Hydrierung des Hydroxypivalinaldehyds erreicht werden kann und dabei sehr hohe Raum-Zeit-Ausbeuten erzielt werden können. Beim Einstellen einer Hydriertemperatur von 80 bis 140°C, vorzugsweise von 110 bis 140°C gelingt die selektive Hydrierung von Hydroxypivalinaldehyd zu Neopentylglykol bei hohen Raum-Zeit-Ausbeuten. Durch die erfindungsgemäße Verwendung von gezielt dotierten Kupferchromit-Katalysatoren sowie durch die genaue Wahl der Hydriertemperatur kann auch die Bildung von Hochsiedern während der Hydrierreaktion unterdrückt werden, im Vergleich zu einer Arbeitsweise, bei der man konventionelle Kupferchromit-Katalysatoren verwendet und bei einer Hydriertemperatur von weniger als 80°C arbeitet. Bei zu niedrigen Hydriertemperaturen wird Hydroxypivalinaldehyd nur unvollständig hydriert. Bei zu hohen Hydriertemperaturen tritt ebenfalls verstärkt eine Zersetzung des als Aldolisierungskatalysator eingesetzten tertiären Alkylamins ein, die zu schwer abtrennbaren Folgeprodukten führt und daher unerwünscht ist. Bei den Hochsiedern handelt es sich um sauerstoffhaltige Verbindungen wie Ester oder cyclische Acetale, in denen Äquivalente des Neopentylglykols chemisch gebunden sind. In den Hochsiedern ist der Anteil an Mono- und Di-iso-Buttersäureestern des Neopentylglykols sowie an dem aus Hydroxypivalinaldehyd nach der Tischtschenko-Reaktion gebildeten Disproportionierungsprodukt Neopentylglykolmonohydroxypivalinsäureester besonders hoch.

Die Aldoladdition von iso-Butyraldehyd und einer wässrigen Formaldehydlösung erfolgt in Gegenwart von tertiären Alkylaminen als Aldoladditionskatalysator, die gleiche oder unterschiedliche Alkylgruppen enthalten können und somit symmetrisch oder unsymmetrisch aufgebaut sein können, sowie in Gegenwart von tertiären Alkylaminen mit mehreren Trialkylaminfunktionen. Beispielsweise wird in Gegenwart von Trimethyl-, Triethyl-, Tri-n-propyl-, Triiso-propyl-, Methyl-diethyl-, Methyl-diisopropylamin, Tri-n-butylamin, Dimethyl-tert-butylamin oder N,N'-Tetramethylethylendiamin gearbeitet. Als besonders geeignete Katalysatoren haben sich Trimethylamin, Triethylamin, Tri-n-propylamin und Tri-n-butylamin bewährt.

Die Aldehyde können im molaren Verhältnis umgesetzt werden, jedoch ist es auch möglich, einen der beiden Reaktionspartner im Überschuss anzuwenden. Man setzt Formaldehyd als wässrige Lösung ein, der Formaldehydgehalt beträgt üblicherweise von 20 bis 50 Gew.-%. Es hat sich herausgestellt, dass der in dem erfindungsgemäßen Verfahren verwendete dotierte Kupfchromit-Katalysator eine überraschend hohe Resistenz gegenüber Formaldehyd besitzt. Daher können in der Aldoladditionsstufe Molverhältnisse von Formaldehyd zu iso-Butyraldehyd von 1:1 bis zu Gunsten von Formaldehyd, im Allgemeinen bis zu 1,2:1, vorzugsweise bis 1,1:1 eingestellt werden. Durch die Verringerung des iso-Butyraldehydeinsatzes wird die iso-Butanolbildung in der Hydrierstufe zurückgedrängt und die Neopentylglykolausbeute, bezogen auf iso-Butyraldehydeinsatz, erhöht.

Die Reaktion zwischen iso-Butyraldehyd und Formaldehyd erfolgt bei Temperaturen zwischen 20 und 100°C, zweckmäßig arbeitet man bei 80 bis 95°C. Im Allgemeinen wird die Reaktion bei Normaldruck durchgeführt, jedoch kann man auch erhöhten Druck anwenden. Das als Aldoladditionskatalysator verwendete tertiäre Alkylamin ist in dem Reaktionsgemisch in einer Menge von 1 bis 20, vorzugsweise 2 bis 12 Mol.-%, bezogen auf iso-Butyraldehyd, enthalten.

Neben dem Wasser aus der wässrigen Formaldehydlösung und geringen Anteilen an Methanol, das ebenfalls in der wässrigen Formaldehydlösung enthalten ist, wird gegebenenfalls der Reaktionsmischung noch iso-Butanol als Verdünnungsmittel zugesetzt. Der iso-Butanolzusatz ist nicht zwingend erforderlich, falls jedoch iso-Butanol zugesetzt wird, liegt sein Gehalt in der Reaktionsmischung im Bereich von 10 bis 20 Gew.-%, bezogen auf das gesamte Reaktionsgemisch. Weitere Lösungs- und Verdünnungsmittel sind nicht erforderlich.

Die praktische Durchführung der Additionsreaktion erfolgt in einem Rührkessel, in einer Rührkesselkaskade oder in einem Reaktionsrohr, das zur besseren Durchmischung der Reaktanten mit Füllkörpern oder anderen Einbauten beschickt sein kann. Die Umsetzung verläuft exotherm, sie kann durch Erhitzen beschleunigt werden.

Das nach der Aldoladdition anfallende Rohgemisch wird gegebenenfalls nach destillativer Abtrennung von flüchtigen Bestandteilen wie Wasser, Methanol, Isobutanol und Restmengen an Formaldehyd, Isobutyraldehyd und gegebenenfalls des Aldolisierungskatalysators oder aber ohne vorherige Trennung in seine Bestandteile oder Abtrennung einzelner Komponenten in Gegenwart des barium- und mangandotierten Kupferchromit-Katalysators katalytisch hydriert.

Vorzugsweise erfolgt die Hydrierung in Gegenwart eines aliphatischen Alkohols, der mit dem Aldolisierungsrohprodukt mischbar ist. Als geeignete aliphatische Alkohole haben sich lineare oder verzweigte Alkohole mit 1 bis 5 Kohlenstoffatomen beispielsweise Methanol, Ethanol, n-Propanol, iso-Propanol, n-Butanol, iso-Butanol, Neopentylglykol oder Mischungen davon erwiesen. Besonders zweckmäßig verwendet man iso-Butanol, da Restmengen von iso-Butyraldehyd zu iso-Butanol hydriert werden. Falls man iso-Butanol bereits in der Aldoladditionsstufe als Verdünnungsmittel einsetzt und nicht vorher destillativ abtrennt, liegt in der Hydrierstufe schon ein Lösungsmittel vor. Geringe Mengen an Methanol, die über die wässrige Formaldehydlösung eingetragen werden, sind ebenfalls zugegen. Der Anteil des aliphatischen Alkohols als organisches Lösungs- oder Verdünnungsmittel beträgt bei dieser Ausgestaltung der Erfindung 15 bis 27 Gew.-%, vorzugsweise 15 bis 23 Gew.-%, bezogen auf den organischen Anteil in dem Einsatzgemisch. Durch den Zusatz des Verdünnungs- oder Lösungsmittels wird eine ausreichende Löslichkeit des Hydroxypivalinaldehyds in der flüssigen Phase während der Hydrierstufe sichergestellt sowie das Ausfallen von Hydroxypivalinaldehyd verhindert und die Homogenität der flüssigen Phase gewährleistet. Bei zu hohen Alkoholgehalten wird wertvolles Reaktorvolumen unnötig belegt und nicht ausgenutzt.

In einer besonders bevorzugten Ausgestaltung des erfindungsgemäßen Verfahrens enthält die für die Hydrierung eingesetzte flüssige Phase neben dem aliphatischen Alkohol auch noch Wasser in einer Menge von 15 bis 25 Gew.-%, vorzugsweise 18 bis 25 Gew.-%, bezogen auf die gesamte Einsatzmenge. Das gesamte, für die Hydrierung verwendete Einsatzgemisch ist homogen und enthält somit 15 bis 25 Gew.-% Wasser und als Rest auf 100 Gew.-% einen organischen Anteil, der wiederum 15 bis 27 Gew.-% eines aliphatischen Alkohols enthält.

Der Wasseranteil fördert eine vorteilhafte Wärmeverteilung und ein vorteilhaftes Abführen der Reaktionswärme bei dem Hydrierschritt und mindert die Gefahr des Auftretens von lokalen Temperaturspitzen.

Das erhaltene, Hydroxypivalinaldehyd enthaltende Rohgemisch wird ohne weitere Reinigungs- und Aufarbeitungsschritte hydriert.

Die Hydrierung des rohen Hydroxypivalinaldehyds wird bei einer Temperatur von 80 bis 140°C, vorzugsweise von 110 bis 140°C, in der flüssigen Phase in Gegenwart von barium- und mangandotierten Kupferchromit-Katalysatoren durchgeführt. Der Reaktionsdruck beträgt 2 bis 18 MPa, vorzugsweise 4 bis 15 MPa. Besonders bewährt sich eine Reaktionstemperatur von 110 bis 140°C und ein Reaktionsdruck von 4 bis 15

MPa. Bei niedrigeren Reaktionsdrücken wird keine zufriedenstellende Hydrierung des Hydroxypivalinaldehyds mehr beobachtet.

Die Hydrierung von Hydroxypivalinaldehyd erfolgt in Gegenwart von Kupferchromit-Katalysatoren, die als Aktivatoren Barium und Mangan enthalten. Kupferchromit-Katalysatoren lassen sich nach H. Adkin, Org. React. 8, 1954, 1-27 als äquimolare Kombination von Kupferoxid und Kupferchromit beschreiben, obgleich sie nicht unbedingt Kupferchromit enthalten. Die Katalysatoren können entweder ohne Trägerstoffe als sogenannte Vollkatalysatoren oder mit Trägerstoffen beispielsweise mit Kieselguhr, Kieselgel oder Aluminiumoxid als Pulver oder in Form von Tabletten, Sternen, Strängen, Ringen oder anderen Teilchen von verhältnismäßig großer Oberfläche verwendet werden.

Zur Herstellung werden entweder unlösliche Verbindungen von Kupfer, Chrom, Mangan und Barium gemischt, beispielsweise in angeteigter Form und zu geeigneten Formkörpern wie Strängen oder Tabletten geformt. Nach der Formgebung wird getrocknet und bis zu 500°C calciniert, wobei sich der Feststoff verdichtet und die anwesenden Metalle gegebenenfalls in die Oxide überführt werden.

Ebenfalls kann man von zweckmäßigerweise wässrigen Lösungen ausgehen, aus denen man das Gelöste ausfällt. Nach Filtration wird der Feststoff getrocknet und wie bei festen Mischungen bis zu 500°C calciniert. Anschließend kann es empfehlenswert sein, den Feststoff in einer niederen organischen Säure wie Ameisensäure, Essigsäure, Propionsäure oder n-Buttersäure aufzurühren um lösliche Bestandteile abzutrennen, anschließend säurefrei zu waschen, erneut zu trocknen und bis zu 500°C zu calcinieren.

Nach Zugabe von Hilfsmitteln wie Graphit oder Alkali- oder Erdalkalimetallseifen können anschließend Formkörper wie Tabletten oder Ringe gefertigt werden.

Die barium- und mangandotierten Kupferchromit-Katalysatoren enthalten von 0,5 bis 8 Gew.-%, vorzugsweise von 3 bis 5 Gew.% Mangan und von 0,5 bis 8 Gew.-%, vorzugsweise von 1 bis 4 Gew.-% Barium, bezogen auf die Summe des Gehalts an Kupfer, Chrom, Barium und Mangan. Besonders bewährt sich ein Bariumgehalt in einem Bereich von 1 bis 4 Gew.-% und ein Mangangehalt in einem Bereich von 3 bis 5 Gew.-%, jeweils bezogen auf die Summe des Gehalts an Kupfer, Chrom, Barium und Mangan. Neben den genannten Aktivatoren können gegebenenfalls noch weitere Aktivatoren wie Cadmium, Magnesium, Strontium und/oder ein seltenes Erdmetall zugegen sein.

Die Hydrierung wird in der Flüssigphase kontinuierlich oder diskontinuierlich durchgeführt, beispielsweise an fest angeordneten Katalysatoren nach der Rieselfahrweise oder Sumpffahrweise sowie nach der Suspensionshydrierung.

Bei der kontinuierlichen Festbettfahrweise hat sich eine Katalysatorbelastung V/Vh, ausgedrückt in Durchsatzvolumen pro Katalysatorvolumen und Zeit, von 0,2 bis 4,0 h$^{-1}$, vorzugsweise 0,8 bis 2,0 h$^{-1}$, als zweckmäßig erwiesen.

Bei diskontinuierlicher Verfahrensführung verwendet man, bezogen auf das flüssige Einsatzprodukt, 1 bis 20, vorzugsweise 2 bis 15 Gew.-% an Kupferchromit-Katalysatoren.

Eine höhere Belastung des Kupferchromit-Katalysators ist zu vermeiden, weil die Ausgangsverbindung Hydroxypivalinaldehyd dann nicht mehr vollständig hydriert wird und eine verstärkte Nebenproduktbildung beobachtet wird.

Vorzugsweise wird die Hydrierung kontinuierlich in flüssiger Phase in einem Rohrreaktor an fest angeordneten Katalysatoren durchgeführt. Unter einem Rohrreaktor ist auch ein Bündel von mehreren eng parallel geschalteten Rohren zu verstehen. Die eingesetzten Rohrreaktoren können ebenfalls Füllkörper oder Einbauten enthalten, beispielsweise Raschigringe, Sättel, Pallringe, Filterplatten oder Kolonnenböden, sowie gegebenenfalls Rührvorrichtungen oder Vorrichtungen zum Abführen der Reaktionswärme. In einer besonders bevorzugten Ausgestaltung erfolgt die Hydrierung von Hydroxypivalinaldehyd in einem Rohrreaktor am Festbett jedoch ohne Einbauten und ohne Rührvorrichtungen.

Die Hydrierung erfolgt vorzugsweise mit reinem Wasserstoff. Es können jedoch auch Gemische verwendet werden, die freien Wasserstoff und daneben unter den Hydrierbedingungen inerte Bestandteile enthalten.

Die Gewinnung des reinen Neopentylglykols aus dem hydrierten Reaktionsgemisch erfolgt nach konventionellen Destillationsverfahren. Dabei abgetrenntes Lösungs- oder Verdünnungsmittel kann wieder in die Aldoladditionsstufe und/oder Hydrierstufe zurückgeführt werden.

Nach dem erfindungsgemäßen Hydrierverfahren wird Hydroxypivalinaldehyd bei hohem Umsatz mit hoher Selektivität und hoher Raum-Zeit-Ausbeute in Neopentylglykol umgewandelt. Bemerkenswert ist der geringe Anteil an Hochsiedern nach Hydrierung und die Bildung von Hochsiedern in der Hydrierstufe kann nachhaltig unterbunden werden. Auch wird die Spaltung des tertiären Alkylamins in flüchtige, stickstoffhaltige Verbindungen, die zu unerwünschten Verunreinigungen führen und die bei der anschließenden destillativen Aufarbeitung nur schwierig abzutrennen sind und die bei der Weiterverarbeitung von Neopentylglykol stören, unterdrückt.

Im Folgenden wird das erfindungsgemäße Verfahren anhand einiger Beispiele näher erläutert.

Beispiele

**Beispiel 1: Herstellen eines bariumdotierten Kupferchromit-Katalysators**

28 g Kupfernitrat-Trihydrat und 2,5 g Bariumnitrat wurden in 200 ml Wasser bei 55°C gelöst. Separat wurden 26 g Ammoniumdichromat in 120 ml Wasser und 40 ml 25%iger Ammoniaklösung gelöst. Danach wurde die Ammoniumdichromatlösung langsam in die Kupfernitratlösung getropft. Dabei fiel ein rot-brauner Feststoff aus. Zur Vervollständigung der Fällung wurde noch eine Stunde nachgerührt und dabei auf Raumtemperatur abgekühlt. Anschließend wurde der Feststoff abfiltriert und bei 110°C im Trockenschrank getrocknet. Der getrocknete Feststoff wurde bei 350°C vier Stunden kalziniert, die Aufheizrate betrug dabei 2°C/min. Nach dem Kalzinieren und Erkalten des Feststoffs wurde dieser mit 200 ml 10%iger Essigsäure aufgerührt. Danach wurde der Feststoff mit Wasser säurefrei gewaschen und erneut bei 110°C getrocknet und bei 350°C mit einer Aufheizrate von 2°C/min geglüht. Der erhaltene Feststoff konnte in dieser Form als Katalysator eingesetzt werden. Auf die Metalle bezogen wies der Katalysator folgende Zusammensetzung auf: 42,1% Kupfer, 48,4% Chrom, 9,5% Barium.

**Beispiel 2: Herstellen eines mangandotierten Kupferchromit-Katalysators**

28 g Kupfernitrat-Trihydrat und 5,0 g Mangannitrat in Form einer 50%igen Lösung in verdünner Salpetersäure wurden in 200 ml Wasser bei 55°C gelöst. Separat wurden 26 g Ammoniumdichromat in 120 ml Wasser und 40 ml 25%iger Ammoniaklösung gelöst. Danach wurde die Ammoniumdichromatlösung langsam in die Kupfernitratlösung getropft. Dabei fiel ein rot-brauner Feststoff aus. Zur Vervollständigung der Fällung wurde noch eine Stunde nachgerührt und dabei auf Raumtemperatur abgekühlt. Anschließend wurde der Feststoff abfiltriert und bei 110°C im Trockenschrank getrocknet. Der getrocknete Feststoff wurde bei 350°C vier Stunden kalziniert, die Aufheizrate betrug dabei 2°C/min. Nach dem Kalzinieren und Erkalten des Feststoffs wurde dieser mit 200 ml 10%iger Essigsäure aufgerührt. Danach wurde der Feststoff mit Wasser säurefrei gewaschen und erneut bei 110°C getrocknet und bei 350°C mit einer Aufheizrate von 2°C/min geglüht. Der erhaltene Feststoff konnte in dieser Form als Katalysator eingesetzt werden. Auf die Metalle bezogen wies der Katalysator folgende Zusammensetzung auf: 50,0% Kupfer, 45,8% Chrom, 4,2% Mangan.

**Beispiel 3: Herstellen eines mangan- und bariumdotierten Kupferchromit-Katalysators**

2,8 kg Kupfernitrat-Trihydrat, 400 g Mangannitrat in Form einer 50%igen Lösung in verdünner Salpetersäure und 150 g Bariumnitrat wurden in 20 Liter Wasser bei 55°C gelöst. Separat wurden 2,6 kg Ammoniumdichromat in 12 Liter Wasser und 4 Liter 25%iger Ammoniaklösung gelöst. Danach wurde die Ammoniumdichromatlösung langsam in die Kupfernitratlösung getropft. Dabei fiel ein rot-brauner Feststoff aus. Zur Vervollständigung der Fällung wurde noch eine Stunde nachgerührt und dabei auf Raumtemperatur abgekühlt. Anschließend wurde der Feststoff abfiltriert und bei 110°C im Trockenschrank getrocknet. Der getrocknete Feststoff wurde bei 350°C vier Stunden kalziniert, die Aufheizrate betrug dabei 2°C/min. Nach dem Kalzinieren und Erkalten des Feststoffs wurde dieser mit 20 Liter 10%iger Essigsäure aufgerührt. Danach wurde der Feststoff mit Wasser säurefrei gewaschen und erneut bei 110°C getrocknet und bei 350°C mit einer Aufheizrate von 2°C/min geglüht. Der erhaltene Feststoff konnte in dieser Form als Katalysator eingesetzt werden. Auf die Metalle bezogen wies der Katalysator folgende Zusammensetzung auf: 47,5% Kupfer, 46,5% Chrom, 4,0% Mangan, 2,0% Barium.

**Beispiel 4: Vergleich der katalytischen Aktivität der Katalysatoren aus den Beispielen 1-3**

Für die Testung der katalytischen Aktivität der gemäß den Beispielen 1-3 hergestellten Kupferchromit-Katalysatoren kam eine rohe Hydroxypivalinaldehydlösung zum Einsatz, die durch Aldolreaktion von iso-Butyraldehyd mit Formaldehyd unter Katalyse von Tri-n-propylamin hergestellt wurde und die folgende Zusammensetzung aufwies: Organischer Anteil (gaschromatographisch ermittelt, Angaben in Prozent):

| | |
|---|---|
| Formaldehyd | 1,4 |
| iso-Butyraldehyd | 7,1 |
| Tri-n-propylamin | 10,3 |
| iso-Butanol | 22,7 |
| Hydroxypivalinaldehyd | 54,4 |
| Neopentylglykol | 0,8 |
| Tischtschenko-Ester | 3,3 |
| | |
| Wasser; in Gew.-% bezogen auf die gesamte Einsatzmischung | 22,4 |

Tischtschenko-Ester: Neopentylglykolmonohydroxypivalinsäureester

Jeweils 10 Massenprozent des Katalysators wurden zur Hydrierung der rohen Hydroxypivalinaldehydlösung eingesetzt.

Die Hydrierung erfolgte in einem Autoklaven bei 130 °C und bei einem Wasserstoffdruck von 8 MPa über vier Stunden. Der Umsatz wurde mit Hilfe folgende Formel ermittelt:

$$\text{Umsatz (\%)} = ((\text{Stoffmenge Hydroxypivalinaldehyd im Ausgangsgemisch} - \text{Stoffmenge Hydroxypivalinaldehyd im Hydrieraustrag}) / \text{Stoffmenge Hydroxypivalinaldehyd im Ausgangsgemisch}) * 100$$

Die Selektivität wurde mit Hilfe folgender Formel ermittelt:

$$\text{Selektivität (\%)} = (\text{Stoffmenge Neopentylglykol im Hydrieraustrag} / (\text{Stoffmenge Hydroxypivalinaldehyd im Ausgangsgemisch} - \text{Stoffmenge Hydroxypivalinaldehyd im Hydrieraustrag})) * 100$$

Dabei wurde folgendes Ergebnis erzielt:

| Katalysator aus Beispiel | 1 | 2 | 3 |
|---|---|---|---|
| Umsatz (%) | 83,3 | 98,4 | 99,4 |
| Selektivität (%) | 91,2 | 94,5 | 94,0 |

**Beispiel 5: Einfluss des tertiären Amins auf die Hydrieraktivität**

10 Massenprozent des Katalysators aus Beispiel 3 wurden zur Hydrierung eines rohen Hydroxypivalinaldehyds folgender Zusammensetzung eingesetzt:

| | |
|---|---|
| Formaldehyd | 1,3 |

(fortgesetzt)

| | |
|---|---|
| iso-Butyraldehyd | 22,0 |
| iso-Butanol | 22,5 |
| Hydroxypivalinaldehyd | 47,1 |
| Neopentylglykol | 5,0 |
| Tischtschenko-Ester | 2,1 |
| | |
| Wasser; in Gew.-% bezogen auf die gesamte Einsatzmischung | 20,3 |

Die Hydrierung erfolgte in einem Autoklaven bei 130°C und bei einem Wasserstoffdruck von 8 MPa über vier Stunden.

Dabei wurde folgendes Ergebnis erzielt:

| | |
|---|---|
| Umsatz (%) | 98,0 |
| Selektivität (%) | 77,9 |

Wie die Umsatz- und besonders die Selektivitätszahl aus dem Beispiel 5 und Beispiel 4/Katalysator 3 zeigt, wirkt sich die Anwesenheit des tertiären Amins in dem rohen Hydroxypivalinaldehyd vorteilhaft auf die Hydriereigenschaften des barium- und mangandotierten Kupferchromit-Katalysators aus.

**Beispiel 6: Einsatz des Katalysators aus Beispiel 3 als Festbettkatalysator**

Der Katalysator aus Beispiel 3 wurde mit 3% Graphit vermischt und tablettiert. Die erhaltenen 3x3 mm Tabletten wurden in einen Rohrreaktor mit 2,2 Liter Volumen gegeben. Anschließend wurde der Katalysator bei 240°C für fünf Stunden in einem 15 mol-% Wasserstoff und 85 mol-% Stickstoff enthaltendem Gasstrom drucklos aktiviert. Hierzu wurden 200 Normliter/h dieses Gasgemisches über den Katalysator geleitet. 1 Normliter bedeuten 1 Liter Gasvolumen bei einer Temperatur von 20°C und bei einem Druck von 0,1 MPa. Rohes Hydroxypivalinaldehyd entsprechend der Zusammensetzung gemäß Beispiel 4 und Wasserstoff wurden am Sumpf des Rohrreaktors kontinuierlich zugeführt. Über den Kopf des Rohrreaktors entnahm man das Hydriergut, leitete es in einen Hochdruckabscheider und führte es daraus über eine Standregelung in eine drucklose Vorlage. Die Hydriertemperatur und die Katalysatorbelastung wurden gemäß den Bedingungen der nachfolgenden Tabelle 1 eingestellt. Der Wasserstoffdruck betrug bei allen Versuchseinstellungen 8 MPa.

Tabelle 1: Kontinuierliche Flüssigphasenhydrierung von Hydroxypivalinaldehyd am mangan- und bariumdotierten Kupferchromit-Katalysator gemäß Beispiel 3

| Temperatur / °C | V/Vh/ h$^{-1}$ | Umsatz Hydroxypivalinaldehyd /% | Selektivität zu Neopentylglykol /% |
|---|---|---|---|
| 155 | 3,00 | 99,7 | 98,9 |
| 145 | 2,00 | 99,8 | 98,9 |
| 135 | 1,75 | 99,9 | 100 |
| 120 | 1,20 | 99,9 | 100 |

Wie die Ergebnisse der Tabelle 1 zeigen, können bei Hydriertemperaturen von 120 bis 135°C selbst bei vergleichsweise hohen Katalysatorbelastungen von 1,2 bis 1,75 Liter Hydriereinsatz pro Liter Katalysator und Stunde ausgezeichnete Umsatz- und Selektivitätswerte und somit hohe Raum-Zeit-Ausbeuten beobachtet werden. Bei noch höheren Temperaturen und Katalysatorbelastungen wird ebenfalls ein nahezu vollständiger Umsatz erzielt allerdings nimmt die Selektivität zum Neopentylglykol ab. Bei diesen, nicht mehr ganz optimalen Versuchseinstellungen ist zudem mit verstärkten Spaltungsreaktionen der als Aldolisierungskatalysator verwendeten tertiären Amine zu rechnen.

**Beispiel 7 (Vergleichsbeispiel):**

Ein kommerzieller, geträgerter Nickelkatalysator in Form von 3x3 mm Tabletten wurde in einen Rohrreaktor mit 2,2 Liter Volumen gegeben. Rohes Hydroxypivalinaldehyd entsprechend der Zusammensetzung gemäß Beispiel 4 und Wasserstoff wurden am Sumpf des Rohrreaktors kontinuierlich zugeführt. Über den Kopf des Rohrreaktors entnahm man das Hydriergut, leitete es in einen Hochdruckabscheider und führte es daraus über eine Standregelung in eine drucklose Vorlage. Die Hydriertemperatur und die Katalysatorbelastung wurden gemäß den Bedingungen der nachfolgenden Tabelle 2 eingestellt. Der Wasserstoffdruck betrug bei allen Versuchseinstellungen 8 MPa.

Tabelle 2: Kontinuierliche Flüssigphasenhydrierung von Hydroxypivalinaldehyd am Nickelkatalysator

| Temperatur / °C | V/Vh/h$^{-1}$ | Umsatz Hydroxypivalinaldehyd /% | Selektivität zu Neopentylglykol /% |
|---|---|---|---|
| 130 | 0,27 | 99,9 | 96,5 |
| 130 | 0,53 | 99,9 | 97,3 |
| 130 | 0,90 | 98,2 | 99,1 |

Wie die Ergebnisse aus Tabelle 2 zeigen, ist an Nickelkatalysatoren nur eine deutlich geringere Raum-Zeit-Ausbeute zu erreichen als an dem Katalysator aus Beispiel 3. Außerdem ist die Selektivität zu Neopentylglykol am Nickelkatalysator niedriger als die am Katalysator aus Beispiel 3.

**Patentansprüche**

1. Verfahren zur Herstellung von Neopentylglykol durch Addition von iso-Butyraldehyd und Formaldehyd in Gegenwart eines tertiären Alkylamins als Katalysator zum Hydroxypivaldehyd mit nachfolgender Hydrierung bei einer Temperatur von 80 bis 140°C und bei einem Druck von 2 bis 18 MPa in der flüssigen Phase, **dadurch gekennzeichnet, dass** die Hydrierung in Gegenwart eines Kupferchromit-Katalysators enthaltend die Aktivatoren Barium und Mangan erfolgt.

2. Verfahren gemäß Anspruch 1, **dadurch gekennzeichnet, dass** die Hydrierung in Gegenwart eines aliphatischen Alkohols in einer Menge von 15 bis 27 Gew.-%, bezogen auf den organischen Anteil in dem Einsatzgemisch, erfolgt.

3. Verfahren gemäß Anspruch 1 oder 2, **dadurch gekennzeichnet, dass** die Hydrierung bei einer Temperatur von 110 bis 140°C und bei einem Druck von 4 bis 15 MPa durchgeführt wird.

4. Verfahren gemäß einem oder mehreren der Ansprüche 1 bis 3, **dadurch gekennzeichnet, dass** man als tertiäres Alkylamin symmetrische tertiäre Alkylamine verwendet.

5. Verfahren gemäß Anspruch 4, **dadurch gekennzeichnet, dass** man als symmetrische tertiäre Alkylamine Trimethylamin, Triethylamin, Tri-n-propylamin oder Tri-n-butylamin verwendet.

6. Verfahren gemäß einem oder mehreren der Ansprüche 1 bis 3, **dadurch gekennzeichnet, dass** man als tertiäres Alkylamin unsymmetrische tertiäre Alkylamine oder Verbindungen mit mehreren Trialkylaminfunktionen verwendet.

7. Verfahren gemäß einem oder mehreren der Ansprüche 2 bis 6, **dadurch gekennzeichnet, dass** man als aliphatischen Alkohol lineare oder verzweigte Alkohole mit 1 bis 5 Kohlenstoffatomen verwendet.

8. Verfahren gemäß Anspruch 7, **dadurch gekennzeichnet, dass** man Methanol, Ethanol, n-Propanol, iso-Propanol, n-Butanol, iso-Butanol, Neopentylglykol oder Mischungen davon verwendet.

9. Verfahren gemäß einem oder mehreren der Ansprüche 1 bis 8, **dadurch gekennzeichnet, dass** der Kupferchromit-Katalysator Barium in einer Menge von 0,5 bis 8 Gew.-%, vorzugsweise von 1 bis 4 Gew.-%, und Mangan in einer Mengen von 0,5 bis 8 Gew.-%, vorzugsweise von 3 bis 5 Gew.-%, jeweils bezogen auf die Summe des Gehalts an Kupfer, Chrom, Barium und Mangen, enthält.

**10.** Verfahren gemäß Anspruch 9, **dadurch gekennzeichnet, dass** der Kupferchromit-Katalysator Barium in einer Menge von 1 bis 4 Gew.-% und Mangan in einer Menge von 3 bis 5 Gew.-%, jeweils bezogen auf die Summe des Gehalts an Kupfer, Chrom, Barium und Mangen, enthält.

**11.** Verfahren gemäß einem oder mehreren der Ansprüche 2 bis 10, **dadurch gekennzeichnet, dass** die für die Hydrierung eingesetzte flüssige Phase Wasser in einer Menge von mehr als 15 bis 25 Gew.-%, vorzugsweise von 18 bis 25 Gew.-%, bezogen auf die gesamte Einsatzmenge, enthält.

**12.** Verfahren gemäß einem oder mehreren der Ansprüche 1 bis 11, **dadurch gekennzeichnet, dass** die Hydrierung in einem Rohrreaktor ohne Einbauten und ohne Rührvorrichtungen durchgeführt wird.

**Claims**

**1.** Method for preparing neopentyl glycol by addition of isobutyraldehyde and formaldehyde in the presence of a tertiary alkylamine as catalyst to give hydroxypivalaldehyde with subsequent hydrogenation at a temperature of 80 to 140°C and at a pressure of 2 to 18 MPa in the liquid phase, **characterized in that** the hydrogenation is carried out in the presence of a copper chromite catalyst comprising the activators barium and manganese.

**2.** Method according to Claim 1, **characterized in that** the hydrogenation is carried out in the presence of an aliphatic alcohol in an amount of 15 to 27% by weight, based on the organic fraction in the starting mixture.

**3.** Method according to Claim 1 or 2, **characterized in that** the hydrogenation is carried out at a temperature of 110 to 140°C and at a pressure of 4 to 15 MPa.

**4.** Method according to one or more of Claims 1 to 3, **characterized in that** the tertiary alkylamines used are symmetrical tertiary alkylamines.

**5.** Method according to Claim 4, **characterized in that** the symmetrical tertiary alkylamines used are trimethylamine, triethylamine, tri-*n*-propylamine or tri-*n*-butylamine.

**6.** Method according to one or more of Claims 1 to 3, **characterized in that** the tertiary alkylamines used are asymmetrical tertiary alkylamines or compounds having a plurality of trialkylamine functions.

**7.** Method according to one or more of Claims 2 to 6, **characterized in that** the aliphatic alcohols used are linear or branched alcohols having 1 to 5 carbon atoms.

**8.** Method according to Claim 7, **characterized in that** methanol, ethanol, *n*-propanol, isopropanol, *n*-butanol, isobutanol, neopentyl glycol or mixtures thereof are used.

**9.** Method according to one or more of Claims 1 to 8, **characterized in that** the copper chromite catalyst comprises barium in an amount of 0.5 to 8% by weight, preferably 1 to 4% by weight, and manganese in an amount of 0.5 to 8% by weight, preferably 3 to 5% by weight, in each case based on the total content of copper, chromium, barium and manganese.

**10.** Method according to Claim 9, **characterized in that** the copper chromite catalyst comprises barium in an amount of 1 to 4% by weight and manganese in an amount of 3 to 5% by weight, in each case based on the total content of copper, chromium, barium and manganese.

**11.** Method according to one or more of Claims to 2 to 10, **characterized in that** the liquid phase used for the hydrogenation comprises water in an amount of more than 15 to 25% by weight, preferably 18 to 25% by weight based on the total amount used.

**12.** Method according to one or more of Claims 1 to 11, **characterized in that** the hydrogenation is carried out in a tubular reactor without internals and without stirring devices.

**Revendications**

1. Procédé pour la préparation de néopentylglycol par addition d'isobutyraldéhyde et de formaldéhyde en présence d'une alkylamine tertiaire en tant que catalyseur, pour l'obtention d'hydroxypivaldéhyde avec hydrogénation subséquente à une température de 80 à 140 °C et sous une pression de 2 à 18 MPa dans la phase liquide, **caractérisé en ce que** l'hydrogénation s'effectue en présence d'un catalyseur à base de chromite de cuivre contenant les activateurs baryum et manganèse.

2. Procédé selon la revendication 1, **caractérisé en ce que** l'hydrogénation s'effectue en présence d'un alcool aliphatique en une quantité de 15 à 27 % en poids, par rapport à la fraction organique dans le mélange de départ.

3. Procédé selon la revendication 1 ou 2, **caractérisé en ce qu'**on effectue l'hydrogénation à une température de 110 à 140 °C et sous une pression de 4 à 15 MPa.

4. Procédé selon une ou plusieurs des revendications 1 à 3, **caractérisé en ce qu'**on utilise comme alkylamine tertiaire des alkylamines tertiaires symétriques.

5. Procédé selon la revendication 4, **caractérisé en ce qu'**on utilise comme alkylamines tertiaires symétriques la triméthylamine, la triéthylamine, la tri-n-propylamine ou la tri-n-butylamine.

6. Procédé selon une ou plusieurs des revendications 1 à 3, **caractérisé en ce qu'**on utilise comme alkylamine tertiaire des alkylamines tertiaires asymétriques ou des composés à plusieurs fonctions trialkylamine.

7. Procédé selon une ou plusieurs des revendications 2 à 6, **caractérisé en ce qu'**on utilise comme alcool aliphatique des alcools linéaires ou des alcools ramifiés ayant de 1 à 5 atomes de carbone.

8. Procédé selon la revendication 7, **caractérisé en ce qu'**on utilise le méthanol, l'éthanol, le n-propanol, l'isopropanol, le n-butanol, l'isobutanol, le néopentylglycol ou des mélanges de ceux-ci.

9. Procédé selon une ou plusieurs des revendications 1 à 8, **caractérisé en ce que** le catalyseur à base de chromite de cuivre contient du baryum en une quantité de 0,5 à 8 % en poids, de préférence de 1 à 4 % en poids, et du manganèse en une quantité de 0,5 à 8 % en poids, de préférence de 3 à 5 % en poids, chaque fois par rapport à la somme de la teneur en cuivre, chrome, baryum et manganèse.

10. Procédé selon la revendication 9, **caractérisé en ce que** le catalyseur à base de chromite de cuivre contient du baryum en une quantité de 1 à 4 % en poids et du manganèse en une quantité de 3 à 5 % en poids, chaque fois par rapport à la somme de la teneur en cuivre, chrome, baryum et manganèse.

11. Procédé selon une ou plusieurs des revendications 2 à 10, **caractérisé en ce que** la phase liquide utilisée pour l'hydrogénation contient de l'eau en une quantité de plus de 15 à 25% en poids, de préférence de 18 à 25 % en poids, par rapport à la quantité de la charge totale de départ.

12. Procédé selon une ou plusieurs des revendications 1 à 11, **caractérisé en ce qu'**on effectue l'hydrogénation dans un réacteur tubulaire sans inserts et sans dispositifs d'agitation.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0278106 A1
- EP 0484800 A2
- US 4855515 A
- WO 9829374 A1
- DE 1518784 A1
- EP 0006460 A1
- GB 2482887 A
- EP 0301853 A1
- DE 102008033163 A1
- EP 0522368 A1

**In der Beschreibung aufgeführte Nicht-Patentliteratur**

- **H. ADKIN.** *Org. React.,* 1954, vol. 8, 1-27